# EUROPÄISCHE PATENTSCHRIFT

(11) **EP 3 031 474 B1**
(45) Veröffentlichungstag und Bekanntmachung des Hinweises auf die Patenterteilung: **13.09.2017**
(21) Anmeldenummer: 15199158.5
(22) Anmeldetag: 10.12.2015
(51) Int. Cl.: A61L 2/07, B67C 3/28

(54) **FÜLLVENTIL FÜR EINEN FÜLLER ZUM BEFÜLLEN EINES BEHÄLTERS MIT EINEM FÜLLPRODUKT**
FILLING VALVE FOR A FILLER FOR FILLING A CONTAINER WITH A FILLING PRODUCT
SOUPAPE DE REMPLISSAGE D'UN RECIPIENT AVEC UN PRODUIT DE REMPLISSAGE

(30) Priorität: 10.12.2014 DE 102014118371
(43) Veröffentlichungstag der Anmeldung: 15.06.2016
(73) Patentinhaber: Krones AG, 93073 Neutraubling (DE)
(72) Erfinder: Poeschl, Stefan, 93073 Neutraubling (DE)
(74) Vertreter: Nordmeyer, Philipp Werner

(56) Entgegenhaltungen:
- EP-A1- 2 604 295
- FR-A- 1 253 805
- FR-A- 1 449 689
- US-A- 3 118 466
- US-A- 3 964 526

## Beschreibung

### Technisches Gebiet

Die vorliegende Erfindung betrifft ein Füllventil für einen Füller zum Befüllen eines Behälters mit einem Füllprodukt, bevorzugt für eine Getränkeabfüllanlage, umfassend einen Ventilsitz und einen dazu komplementären Ventilkegel, der zur Bereitstellung einer Abdichtung in den Ventilsitz absenkbar ist. Die vorliegende Erfindung betrifft weiterhin ein Verfahren zum Sterilisieren eines Füllers.

### Stand der Technik

Es ist bekannt, zumindest die produktführenden Kanäle und Teile einer Getränkeabfüllanlage und insbesondere eines Füllventils einer Getränkeabfüllanlage in einem geschlossenen Reinigungskreislauf zu reinigen. Dazu wird beispielsweise eine Produktabgabeöffnung eines Füllventils mittels einer sogenannten CIP (cleaning in place) Kappe verschlossen und das Reinigungsmedium entsprechend in einem Kreislauf geführt. Dadurch können produktberührte Anlagenteile im Inneren der Getränkeabfüllanlage ohne Demontage gereinigt werden.

Gleichermaßen ist es bekannt, nach der Reinigung auch eine Sterilisierung zumindest der produktberührten Anlagenteile mittels eines Sterilisationsmediums durchzuführen, um eine Sterilisation der Anlage vor der Aufnahme des nächsten Füllbetriebs zu erreichen. Als Sterilisationsmedium wird beispielsweise Heißdampf, bevorzugt Sattdampf, verwendet. Mittels des Heißdampfes werden zumindest die produktberührten Oberflächen auf die für die Sterilisation erforderliche Temperatur gebracht und für einen für die Sterilisation erforderlichen Zeitraum auf dieser Temperatur gehalten.

Um die Sterilisation durchführen zu können, werden üblicherweise die CIP Kappen, welche die Produktabgabeöffnungen des Füllventils verschließen, durch Sterilisationskappen ausgetauscht. Die Sterilisationskappen weisen eine Bohrung auf, durch welche hindurch Kondensat abfließen kann, um eine vollständige Sterilisierung aller Oberflächen zu erreichen. Eventuell sich auf und über der Sterilisationskappe ansammelndes und im Füllventil ansteigendes Kondensat würde eine zuverlässige Beaufschlagung der in diesem Bereich vorliegenden Oberflächen mit dem Sterilisationsmedium verhindern, so dass das Kondensat zum Erreichen einer ordnungsgemäßen Sterilisation abgeleitet werden muss.

Durch die Bohrung kann ferner Dampf austreten, wodurch zum einen das Vorliegen eines steten Dampfstroms durch das Füllventil hindurch erreicht wird, so dass Temperatur und Menge des Sterilisationsmediums erhalten bleiben, und zum anderen der Austritt des Dampfes auch über entsprechende Sensoren erfasst werden kann, um den Ablauf der Sterilisation des Füllorgans, insbesondere des Füllventils, zu überwachen.

Ein Nachteil der Sterilisierung mittels einer Sterilisationskappe liegt in dem erforderlichen Bauraum durch zusätzliche Schwenkvorrichtungen, Antriebe und Steuerungen, welche für die Bereitstellung und Betätigung der Sterilisationskappe vorgesehen werden müssen. Diese zusätzlichen Komponenten stellen in einer Getränkeabfüllanlage auch zusätzliche Anforderungen an Reinigung, Sterilisierung und hygienisches Design.

Die US 3,118,466 A beschreibt ein mit Druck zu betätigendes Umgehungsventil für eine Pumpvorrichtung. Die EP 2 604 295 A1 beschreibt eine Vorrichtung zum Sterilisieren von Behältnissen mit Ableitungselement für Sterilisationsmittel. Die US 3,964,526 A beschreibt ein Verfahren und eine Vorrichtung zum Reinigen von rotierenden Abfüllmaschinen. Die FR 1.449.689 A und FR 1.253.805 A beschreiben Verfahren und Vorrichtungen zum Füllen von Behältern.

### Darstellung der Erfindung

Ausgehend von dem bekannten Stand der Technik ist es eine Aufgabe der vorliegenden Erfindung, ein verbessertes Füllventil sowie ein verbessertes Verfahren zum Sterilisieren eines Füllers bereitzustellen.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird ein Füllventil für einen Füller zum Befüllen eines Behälters mit einem Füllprodukt, bevorzugt für eine Getränkeabfüllanlage, angegeben, welches einen Ventilsitz und einen dazu komplementären Ventilkegel, der zur Bereitstellung einer Abdichtung in den Ventilsitz absenkbar ist, umfasst. Erfindungsgemäß ist dabei ein Fluidbypass zur Überbrückung der durch die Absenkung des Ventilkegels in den Ventilsitz bereitgestellten Abdichtung für ein Fluid bereitgestellt. Ferner ist ein Füllproduktzulaufventil stromaufwärts des Ventilsitzes angeordnet, das eingerichtet ist, um einen Zulauf von Füllprodukt zu dem Ventilsitz im Füllbetrieb zu unterbrechen.

Dadurch ist es möglich, das Füllventil ohne die Bereitstellung einer Sterilisationskappe zu sterilisieren. Vor der Sterilisierung des Füllventils muss der Ventilkegel lediglich in den Ventilsitz abgesenkt werden. Während der Sterilisierung wird ein Füllorganinnenraum mit einem Sterilisationsmedium beaufschlagt. Das Sterilisationsmedium wird dabei unter einem vorgegebenen Druck und einer vorgegebenen Temperatur durch den Füllorganinnenraum geführt, wobei der Druck und damit die Temperatur aufrechterhalten bleibt, da das Ventil im Wesentlichen geschlossen ist und durch den Fluidbypass wie eine herkömmliche Sterilisationskappe wirkt. Als Sterilisationsmedium kommt in der Regel Sattdampf zum Einsatz. Mittels des Fluidbypasses ist es möglich, das sich während der Sterilisierung im Füllorganinnenraum sammelnde Kondensat abzuführen und gleichzeitig auch bei Erhaltung des Drucks für einen konstanten Volumenstrom des Sattdampfs durch das Füllventil hindurch zu sorgen.

Durch das Abführen des Kondensats können in dem Füllorganinnenraum alle Oberflächen mit dem Sterilisationsmedium in Kontakt kommen und so sterilisiert werden. Ein Abdecken von Oberflächenbereichen durch das Kondensat wird so unterbunden. Weiterhin können gemeinsam mit dem Kondensat auch im Füllorganinnenraum befindliche Fremdstoffpartikel, welche die Haltbarkeit eines Füllprodukts reduzieren und bei kohlensäurehaltigen Getränken zu einem Entlösen von Kohlenstoffdioxid führen könnten, entfernt werden.

Darüber hinaus ermöglicht der Fluidbypass, dass während der Sterilisierung Dampf durch den Fluidbypass aus dem Füllorganinnenraum austreten kann, wobei der dabei austretende Volumenstrom für einen steten Fluss an Sterilisationsmedium durch den Füllorganinnenraum hindurch sorgt. Weiterhin kann der Austritt des Dampfes gemessen werden, um den Ablauf der Sterilisierung des Füllorganinnenraums zu überwachen.

Um zu verhindern, dass jedoch während eines Füllvorgangs in dem Füllorganinnenraum befindliches Füllprodukt bei einem in den Ventilsitz abgesenkten Ventilkegel durch den stets offenen Fluidbypass ausläuft, ist ein Füllproduktzulauf, durch welchen das Füllprodukt dem Füllventil zugeführt wird, mit einem Füllproduktzulaufventil versehen, welches den Füllproduktzulauf vollständig schließen kann. Ist der Füllproduktzulauf geschlossen und der Ventilkegel zur Bereitstellung einer Abdichtung des Füllventils in den Ventilsitz abgesenkt, kann das Füllprodukt immer noch in den Fluidbypass eintreten und je nach Druckverhältnissen und Viskosität des Füllprodukts auch aus diesem ausfließen. Ein solches Ausfließen würde zu einer erhöhten Tropfneigung des Füllventils und Ungenauigkeiten beim Füllvorgang führen. Durch das Verschließen des Füllproduktzulaufventils kann aber im Füllorganinnenraum ein Unterdruck bereitgestellt werden, so dass das Füllprodukt bei in den Ventilsitz abgesenktem Ventilkegel nicht durch den Fluidbypass ausfließen kann. Somit wird der reguläre Füllbetrieb nicht durch die Bereitstellung des Fluidbypasses beeinträchtigt.

Dadurch, dass keine Sterilisationskappe zur Sterilisierung des Füllers benötigt wird, wird auch keine Schwenkeinheit mit Antrieb und Steuerung benötigt, mittels welcher diese Sterilisationskappe vor der Sterilisierung auf das Füllventil geschwenkt werden würde. Daraus ergeben sich geringere Materialkosten sowie ein geringerer Platzbedarf bei der Bereitstellung einer Sterilisierung in einem geschlossenen Kreislauf. Durch den Wegfall einer Schwenkbewegung wird die für die Sterilisierung benötigte Zeit verkürzt und eine Sterilisierung kann im Prinzip unmittelbar anschließend an einen Füllvorgang durchgeführt werden.

In einer bevorzugten Ausführungsform ist der Ventilsitz zwischen einem Füllorganinnenraum und einer Produktabgabeöffnung angeordnet und der Fluidbypass verbindet den Füllorganinnenraum bei abdichtend abgesenktem Ventilkegel mit der Produktabgabeöffnung.

Dadurch wird eine Verbindung von dem Füllorganinnenraum zur Umgebung des Füllers bereitgestellt. Somit ermöglicht der Bypass die Ausscheidung von während der Sterilisierung entstehendem Kondensat vom Füllorganinnenraum zur Produktabgabeöffnung und somit zur Umgebung des Füllers. Ferner können im Bereich der Produktabgabeöffnungen Messungen von aus dem Fluidbypass ausströmendem Dampf durchgeführt werden, um die Sterilisationsfunktion des entsprechenden Füllers zu überwachen.

Darüber hinaus können durch die fluidtechnische Verbindung des Füllorganinnenraums mit der Produktabgabeöffnung mittels des Fluidbypasses Druckspitzen, welche durch das Sterilisationsmedium im Füllorganinnenraum hervorgerufen werden, abgebaut werden.

In einer bevorzugten Weiterbildung weist der Fluidbypass die Form einer Bohrung auf. Dadurch weist der Fluidbypass die Eigenschaften einer engen Röhre auf, durch welche der Kapillareffekt begünstigt wird. Dieser ist unter anderem nötig, um zu verhindern, dass Füllprodukt während des Füllvorgangs durch den Fluidbypass austritt. Darüber hinaus lässt sich ein Bypass in Form einer Bohrung einfach herstellen. Somit können bereits bestehende Füller auch einfach nachgerüstet werden.

Ein Fluidbypass in Form einer Bohrung weist kaum beziehungsweise keine Ecken auf, in welchen sich Rückstände bilden können. Entsprechend ist ein derartiger Fluidbypass auch aus hygienischer Sicht vorteilhaft.

In einer weiter bevorzugten Ausführungsform weist der Fluidbypass einen Durchmesser oder eine Breite von 0,1 mm bis 5 mm, bevorzugt 0,5 mm bis 4 mm, und besonders bevorzugt 1 mm bis 3 mm, auf.

Dadurch weist der Fluidbypass die Form einer engen Röhre auf, wodurch das Austreten des Füllprodukts bei geschlossenem Füllventil vermieden werden kann, wobei gleichzeitig unter dem hohen Druck während der Sterilisierung mit Sattdampf Kondensat durch den Fluidbypass herausgedrängt wird und ein konstanter Volumenstrom an Sterilisationsmedium durch das Füllventil hindurch erreicht wird.

Während eines Füllvorgangs wirkt der Fluidbypass entsprechend wie eine Kapillare, wobei Füllprodukt vom Füllorganinnenraum in den Fluidbypass eintritt, bis es schließlich durch den bei geschlossenem Füllproduktzulaufventil im Füllorganinnenraum herrschenden Unterdruck zurückgehalten wird. Somit verhindert der Unterdruck im Füllorganinnenraum, dass das Füllprodukt durch den Fluidbypass ausläuft.

Darüber hinaus kann durch einen derartigen Fluidbypass sichergestellt werden, dass nur eine begrenzte Menge an Sterilisationsmedium, wie beispielsweise Sattdampf, aus dem Füllorganinnenraum entweichen kann, so dass im Füllorganinnenraum ein ausreichend hoher Druck zur Sterilisierung bereitgestellt werden kann. Die Abmessungen des Fluidbypasses ermöglichen ferner, dass während der Sterilisierung auftretendes Kondensat und auftretender Dampf auch bei im Füllorganinnenraum herrschenden hohen Drücken durch den Fluidbypass austreten kann.

Der Fluidbypass ist bevorzugt so dimensioniert und ausgestaltet, dass er bei den relativ niedrigen Drücken im Abfüllbetrieb ein Austreten des Füllprodukts verhindert, bei den relativ hohen Drücken des Sterilisationsbetriebs hingegen ein Austreten des Sterilisationsmediums und des Kondensats ermöglicht.

In einer bevorzugten Weiterbildung ist der Fluidbypass in dem Ventilkegel angeordnet. Dadurch beschränkt sich die Nachrüstung für herkömmliche Systeme lediglich auf die Anpassung des Ventilkegels. Beispielsweise kann ein herkömmlicher Ventilkegel um eine Bohrung ergänzt werden, welche bei abdichtend abgesenktem Ventilkegel den Füllorganinnenraum mit der Produktabgabeöffnung verbindet.

Die Bereitstellung des Fluidbypasses im Ventilkegel erfordert keinen zusätzlichen Materialaufwand. Vielmehr ist es möglich, gegenüber der Bereitstellung einer herkömmlichen Sterilisierung, beispielsweise unter Verwendung einer Sterilisationskappe, Material einzusparen.

In einer bevorzugten Weiterbildung ist der Fluidbypass auf einer Dichtkontaktoberfläche des Ventilkegels in Form einer Aussparung, bevorzugt einer Nut, und besonders bevorzugt einer Nut mit einem abgerundeten Boden, angeordnet.

Die Dichtkontaktoberfläche des Ventilkegels ist die Oberfläche, welche mit dem Ventilsitz in Kontakt kommt, wenn der Ventilkegel abdichtend in diesen abgesenkt ist. Steht die Dichtkontaktfläche des Ventilkegels mit dem Ventilsitz in abdichtendem Kontakt, kann das Sterilisationsmedium lediglich durch den Fluidbypass vom Füllorganinnenraum zur Produktabgabeöffnung strömen beziehungsweise den in den Ventilsitz abgesenkten Ventilkegel überbrücken.

Durch Anordnen des Fluidbypasses auf der Oberfläche des Ventilkegels kann die Unversehrtheit des Fluidbypasses einfach überprüft werden. Die Kontrolle der Oberfläche des Ventilkegels reicht aus, um mögliche Rückstände, beispielsweise Füllproduktrückstände, in dem Fluidbypass zu erkennen.

Die Bereitstellung des Fluidbypasses auf der Oberfläche des Ventilkegels hat zur Folge, dass, wenn der Ventilkegel nicht in den Ventilsitz abgesenkt ist, der Fluidbypass Teil der übrigen Oberfläche des Ventilkegels wird. Wird der Ventilkegel entsprechend aus dem Ventilsitz herausgehoben, vereint sich die Aussparung auf der Oberfläche des Ventilkegels mit dem Spalt, welcher aufgrund des Anhebens des Ventilkegels zwischen der Dichtkontaktoberfläche des Ventilkegels und der Oberfläche des Ventilsitzes entsteht. Entsprechend ist der Fluidbypass nur dann bereitgestellt, wenn der Ventilkegel abdichtend in den Ventilsitz abgesenkt ist.

Es ist möglich, den Ventilkegel je nach gewünschter Ausführung des Fluidbypasses gegen einen anderen mit einer anderen Geometrie oder Ausbildung des Fluidbypasses auszutauschen.

In einer weiter bevorzugten Ausgestaltung ist der Fluidbypass in dem Ventilsitz angeordnet. Dadurch beschränkt sich die Nachrüstung für herkömmliche Systeme lediglich auf die Anpassung des Ventilsitzes. Beispielsweise kann ein herkömmlicher Ventilsitz um eine Bohrung ergänzt werden, welche bei abdichtend abgesenktem Ventilkegel den Füllorganinnenraum mit der Produktabgabeöffnung verbindet.

In einer weiter bevorzugten Ausführungsform ist der Fluidbypass auf einer Dichtkontaktoberfläche des Ventilsitzes in Form einer Aussparung, bevorzugt einer Nut, und besonders bevorzugt einer Nut mit einem abgerundeten Boden, angeordnet.

Die Dichtkontaktoberfläche des Ventilsitzes ist die Oberfläche, welche mit dem Ventilkegel in Kontakt kommt, wenn der Ventilkegel auf sie abgesenkt worden ist. Steht die Dichtkontaktfläche des Ventilsitzes mit dem Ventilkegel in abdichtendem Kontakt, kann das Sterilisationsmedium lediglich durch den Fluidbypass vom Füllorganinnenraum zur Produktabgabeöffnung strömen beziehungsweise den auf den Ventilsitz abgesenkten Ventilkegel überbrücken.

Durch Anordnen des Fluidbypasses auf der Oberfläche des Ventilsitzes kann die Unversehrtheit des Fluidbypasses einfach überprüft werden. Die bloße Kontrolle der Oberfläche des Ventilsitzes reicht aus, um mögliche Rückstände, beispielsweise Füllproduktrückstände, in dem Fluidbypass zu erkennen.

Die Bereitstellung des Fluidbypasses auf der Oberfläche des Ventilsitzes hat zur Folge, dass, wenn der Ventilkegel nicht in den Ventilsitz abgesenkt ist, der Fluidbypass eins mit der übrigen Oberfläche des Ventilkegels wird. Wird der Ventilkegel aus dem Ventilsitz herausgehoben, vereint sich die Aussparung auf der Oberfläche des Ventilsitzes mit dem Spalt, welcher aufgrund des Anhebens des Ventilkegels zwischen der Dichtkontaktoberfläche des Ventilkegels und der Oberfläche des Ventilsitzes entsteht. Entsprechend ist der Fluidbypass nur dann bereitgestellt, wenn der Ventilkegel abdichtend in den Ventilsitz abgesenkt ist.

Die oben genannte Aufgabe wird auch durch ein Verfahren mit den Merkmalen des Anspruchs 9 gelöst. Vorteilhafte Ausgestaltungen ergeben sich aus den Unteransprüchen.

Entsprechend wird ein Verfahren zum Sterilisieren eines Füllers, welcher mindestens ein Füllventil gemäß einem der vorstehenden Aspekte umfasst, angegeben. Dabei umfasst das Verfahren die Schritte des Absenkens des Ventilkegels auf den Ventilsitz und des Beaufschlagens des Füllers, bevorzugt eines Füllorganinnenraums, mit einem Sterilisationsmedium. Erfindungsgemäß überbrückt dabei während der Sterilisierung des Füllers das Sterilisationsmedium durch den Fluidbypass die durch die Absenkung des Ventilkegels in den Ventilsitz bereitgestellte Abdichtung.

Dadurch ist es möglich, das Füllventil ohne die Bereitstellung einer Sterilisationskappe zu sterilisieren. Vor der Sterilisierung des Füllventils muss der Ventilkegel lediglich in den Ventilsitz abgesenkt werden. Während der Sterilisierung wird ein Füllorganinnenraum mit einem Sterilisationsmedium beaufschlagt. Das Sterilisationsmedium wird dabei unter einem vorgegebenen Druck und einer vorgegebenen Temperatur durch den Füllorganinnenraum geführt. Als Sterilisationsmedium kommt dabei in der Regel Sattdampf zum Einsatz. Mittels des Fluidbypasses ist es möglich, sich während der Sterilisierung im Füllorganinnenraum sammelndes Kondensat abzuführen.

Darüber hinaus ermöglicht der Fluidbypass, dass während der Sterilisierung Dampf durch den Fluidbypass aus dem Füllorganinnenraum austreten kann, wobei der Austritt des Dampfes gemessen werden kann, um den Ablauf der Sterilisierung des Füllorganinnenraums zu überwachen.

In einer Weiterbildung wird das Sterilisationsmedium mit einem Druck von 1,5 bar bis 5,5 bar, bevorzugt 2 bar bis 5 bar, besonders bevorzugt 3 bar bis 4 bar, und ganz besonders bevorzugt 3,5 bar bei einer Temperatur zwischen 100°C und 150°C, bevorzugt 121°C bis 135°C, bereitgestellt.

Dadurch können je nach verwendetem Sterilisationsmedium die geeigneten Sterilisationsbedingungen bereitgestellt werden und die zu sterilisierenden Oberflächen auf die jeweils gewünschte Sterilisationstemperatur gebracht werden.

Dadurch, dass das Sterilisationsmedium mit einer Temperatur zwischen 100°C und 150°C im Füllorganinnenraum bereitgestellt wird, ist es möglich, die Dichtkontaktoberflächen des Ventilsitzes und des Ventilkegels auch dann zu sterilisieren, wenn der Ventilkegel abdichtend in den Ventilsitz abgesenkt ist. Dazu sind der Ventilsitz und/oder der Ventilkegel typischerweise aus Materialien mit einer Wärmeleitfähigkeit bereitzustellen, so dass die von dem Sterilisationsmedium ausgehende Temperatur im Füllorganinnenraum sich bei in den Ventilsitz abgesenktem Ventilkegel über die Dichtkontaktoberflächen des Ventilsitzes und/oder des Ventilkegels ausbreiten kann.

In einer weiter bevorzugten Ausgestaltung wird der Ventilkegel zum Sterilisieren von Dichtkontaktoberflächen des Ventilsitzes und des Ventilkegels aus dem Ventilsitz herausgehoben, bevorzugt periodisch oder taktweise, und beim Eintreten eines vorbestimmten Ereignisses wieder in den Ventilsitz abgesenkt. Bei mehreren nebeneinander angeordneten Füllventilen in einem Füller kann es vorteilhaft sein, die unterschiedlichen Füllventile abwechselnd zu öffnen, so dass durch das Öffnen erzeugten Druckschwankungen reduziert werden.

Dadurch werden die Dichtkontaktoberflächen des Ventilsitzes und des Ventilkegels unmittelbar mit Sterilisationsmedium beaufschlagt. Somit werden neben dem Füllorganinnenraum auch die Dichtkontaktoberflächen des Ventilsitzes und des Ventilkegels sterilisiert. Durch ein periodisches oder taktweises Herausheben des Ventilkegels aus dem Ventilsitz ist es möglich, dass sich im Füllorganinnenraum bei in den Ventilsitz abgesenktem Ventilkegel die für die Sterilisierung notwendigen Druckverläufe und Temperaturverläufe einstellen. Wird der Ventilkegel aus dem Ventilsitz herausgehoben, strömt Sterilisationsmedium über die Dichtkontaktflächen des Ventilsitzes und des Ventilkegels aus dem Füllorganinnenraum heraus, was eine Abnahme des Druckes sowie der Temperatur im Füllorganinnenraum zur Folge hat. Anschließend wird der Ventilkegel wieder in den Ventilsitz abgesenkt, so dass sich im Füllorganinnenraum wieder ein vorgegebener Druck und eine vorgegebene Temperatur des Sterilisationsmediums einstellen können.

Alternativ kann das Herausheben des Ventilkegels aus dem Ventilsitz derart kurzzeitig erfolgen, dass der Druck und die Temperatur des Sterilisationsmediums im Füllorganinnenraum nahezu unverändert bleiben. Das periodische Herausheben des Ventilkegels aus dem Ventilsitz hat dann eine schrittweise Sterilisierung der Dichtkontaktoberflächen des Ventilsitzes und des Ventilkegels zur Folge.

In einer bevorzugten Ausführungsform ist das vorbestimmte Ereignis ein Ablaufen einer vorbestimmten Zeit und/oder das Erreichen einer vorbestimmten Temperatur der Dichtkontaktoberfläche des Ventilsitzes und/oder der Dichtkontaktoberfläche des Ventilkegels.

Bildet das vorbestimmte Ereignis den Ablauf einer vorbestimmten Zeit, so kann die Zeit, für welche der Ventilkegel aus dem Ventilsitz herausgehoben ist, beispielsweise nach der Zeit bestimmt sein, die es benötigt, um die Dichtkontaktoberflächen des Ventilsitzes und des Ventilkegels mittels aus dem Füllorganinnenraum ausströmenden Sterilisationsmediums auf die für die Sterilisation erforderliche Temperatur zu erhöhen und damit die Dichtkontaktoberfläche zu sterilisieren. Alternativ kann die Zeitspanne, in welcher der Ventilkegel aus dem Ventilsitz herausgehoben ist, sich auch an der Bereitstellung eines nahezu konstanten Druckes und einer nahezu konstanten Temperatur im Füllorganinnenraum orientieren.

Stellt das Ereignis, nach dessen Eintritt der Ventilkegel wieder in den Ventilsitz abgesenkt wird, das Erreichen einer vorbestimmten Temperatur der Dichtkontaktoberfläche des Ventilsitzes und/oder der Dichtkontaktoberfläche des Ventilkegels dar, ist es nötig, die Temperatur an den Dichtkontaktoberflächen über entsprechende Sensoren zu messen. Wird beispielsweise festgestellt, dass die Dichtkontaktoberflächen eine vorbestimmte Temperatur erreichen, während der Ventilkegel aus dem Ventilsitz herausgehoben ist und das Sterilisationsmedium über die Dichtkontaktflächen strömt, kann der Ventilkegel wieder in den Ventilsitz abgesenkt werden. Die vorbestimmte Temperatur ist beispielsweise eine Temperatur, bei welcher sichergestellt ist, dass die Dichtkontaktoberflächen eine ausreichende Sterilisierung erfahren haben.

In einer Weiterbildung wird der Ventilkegel zum Sterilisieren von Dichtkontaktoberflächen des Ventilsitzes und des Ventilkegels nur um 1 mm bis 3 mm aus dem Ventilsitz angehoben.

Dadurch ist es möglich, die Dichtkontaktoberflächen des Ventilsitzes und des Ventilkegels mit dem Sterilisationsmedium zu beaufschlagen. Gleichzeitig kann durch ein Anheben des Ventilkegels um maximal 3 mm aus dem Ventilsitz verhindert werden, dass sich der Druck und die Temperatur im Füllorganinnenraum aufgrund des Anhebens des Ventilsitzes stark reduzieren. Entsprechend können die Sterilisationsbedingungen im Füllorganinnenraum beibehalten werden.

Bevorzugt ist ein Füllproduktzulaufventil stromaufwärts der Abdichtung angeordnet und im Füllbetrieb wird ein Zulauf von Füllprodukt bei in den Ventilsitz abgesenktem Ventilkegel durch das Füllproduktzulaufventil unterbrochen. Auf diese Weise kann ein Nachtropfen beziehungsweise ein Austreten von Füllprodukt durch den Fluidbypass bei geschlossenem Füllventil verhindert werden.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: schematisch eine Schnittansicht eines Füllorgans mit einer Bohrung durch den Ventilkegel,
- Figur 2: schematisch eine Schnittansicht des Füllorgans der vorstehenden Figur entlang des in Figur 1 gezeigten Schnitts A-A,
- Figur 3: schematisch eine Schnittansicht eines Füllventils, wobei auf der Oberfläche des Ventilkegels eine Nut verläuft,
- Figur 4: schematisch eine Schnittansicht der vorstehenden Figur entlang des in Figur 3 gezeigten Schnitts B-B,
- Figur 5: schematisch ein Füllventil, wobei im Ventilsitz eine Bohrung angeordnet ist,
- Figur 6: schematisch eine Schnittansicht des Füllventils der vorstehenden Figur entlang des in Figur 5 gezeigten Schnitts C-C,
- Figur 7: schematisch eine Schnittansicht eines Füllventils, wobei im Ventilsitz eine Nut verläuft, und
- Figur 8: schematisch eine Schnittansicht des Füllventils der vorstehenden Figur entlang des in Figur 7 gezeigten Schnitts D-D.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf eine wiederholte Beschreibung dieser Elemente in der nachfolgenden Beschreibung teilweise verzichtet.

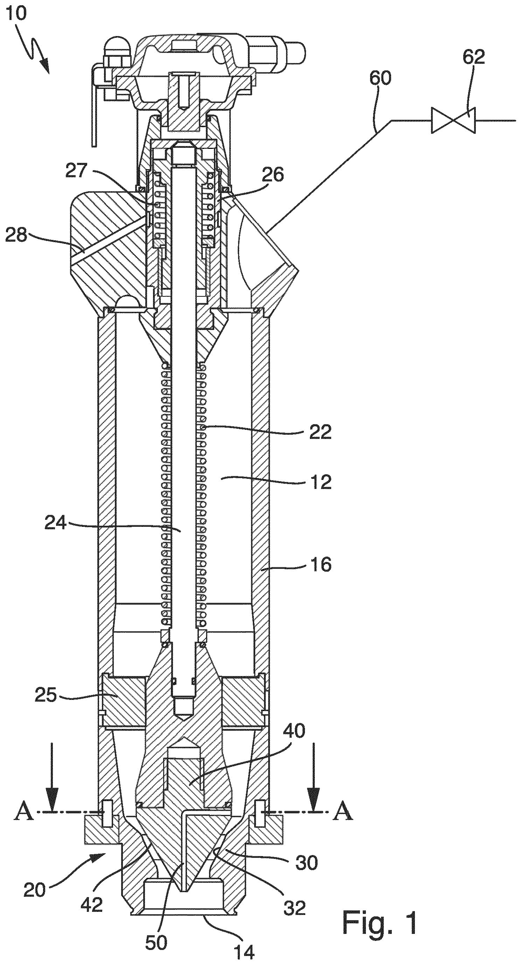

Figur 1 ist ein Füllorgan 10 zum Befüllen eines Behälters mit einem Füllprodukt zu entnehmen. Das Füllorgan 10 ist in einem oberen Bereich an einen Füllproduktzulauf 60 angeschlossen, durch welchen dem Füllorgan 10 Füllprodukt von einem nicht gezeigten Produktkessel zugeführt werden kann. Die Füllproduktzufuhr 60 umfasst ein Füllproduktzulaufventil 62, mittels welchem der Füllproduktzulauf 60 geöffnet und geschlossen werden kann. Entsprechend kann über das Füllproduktzulaufventil 62 die Zufuhr von Füllprodukt an das Füllventil 10 bereitgestellt beziehungsweise unterbrochen werden. Darüber hinaus lässt sich mittels des Füllproduktzulaufventils 62 die Menge des dem Füllorgan 10 über den Füllproduktzulauf 60 zugeführten Füllproduktes pro Zeiteinheit steuern.

Das Füllorgan 10 weist ein rohrförmiges Gehäuse 16 auf, welches einen Füllorganinnenraum 12 von der Umgebung trennt. Durch den Füllproduktzulauf 60 zugeführtes Füllprodukt kann in dem Füllorganinnenraum 12 aufgenommen werden. Das Füllorgan 10 weist an einem unteren Ende eine Produktabgabeöffnung 14 auf, welche dazu dient, Füllprodukt aus dem Füllorgan 10 in einen Behälter abzugeben.

Zwischen dem Füllorganinnenraum 12 und der Produktabgabeöffnung 14 ist ein Ventilkegel 40 angeordnet. Die Oberfläche des Ventilkegels 40 verläuft teilweise komplementär zu der Oberfläche eines Ventilsitzes 30, welcher im unteren Teil des Gehäuses 16 angeordnet ist. In dem komplementären Bereich bildet sich bei in dem Ventilsitz 30 aufgenommenem Ventilkegel 40 eine Abdichtung aus.

Der Ventilkegel 40 kann auf den Ventilsitz 30 abgesenkt werden, so dass der Füllorganinnenraum 12 gegenüber der Produktabgabeöffnung 14 abgedichtet ist. Zur Bereitstellung einer Füllproduktzufuhr zu einem Behälter zu dessen Befüllung wird der Ventilkegel 40 aus dem Ventilsitz 30 herausgehoben, so dass Füllprodukt zwischen dem Ventilkegel 40 und dem Ventilsitz 30 hindurch aus dem Füllorganinnenraum 12 zur Produktabgabeöffnung 14 fließen kann.

Das Anheben und Absenken des Ventilkegels 40 erfolgt über einen Hubzylinder 62, welcher über eine Ventilstange 24 mit dem Ventilkegel 40 verbunden ist. Zur Erzeugung einer Hubbewegung des Ventilkegels 40 kann über einen Druckluftkanal 28 Luft in den Hubzylinder 26 hinein oder aus dem Hubzylinder 26 heraus strömen. Zur Unterstützung der Hubbewegung ist die Ventilstange 24 über Federn 22, 27 vorgespannt.

Um sicherzustellen, dass eine Dichtkontaktoberfläche 42 des Ventilkegels 40 unter Ausbildung einer Abdichtung abdichtend auf eine Dichtkontaktoberfläche 32 des Ventilsitzes 30 abgesenkt wird, wird der Ventilkegel 40 beziehungsweise die Ventilstange 24 von einer Führung 25 axial geführt. Die Führung 25 ist dabei im unteren Bereich des Gehäuses 16 angeordnet.

Der Ventilkegel 40 weist einen Fluidbypass 50 auf, welcher im gezeigten Ausführungsbeispiel durch eine horizontale und eine senkrechte Bohrung im Ventilkegel 40 gebildet ist. Der Fluidbypass 50 ermöglicht eine fluidtechnische Verbindung des Füllorganinnenraumes 12 mit der Produktabgabeöffnung 14, gerade auch wenn der Ventilkegel 40 in den Ventilsitz 30 abdichtend abgesenkt ist.

Um zu verhindern, dass im Füllbetrieb bei in den Ventilsitz 30 abgesenktem Ventilkegel 40 Füllprodukt durch den Fluidbypass 50 aus dem Füllorgan 10 austreten kann, wird das Füllproduktzulaufventil 62 geschlossen. Das Füllprodukt, welches sich in dem Fluidbypass 50 befindet, verschließt den Fluidbypass 50 entsprechen derart, dass kein Fluid, beispielsweise Gas, aus der Umgebung durch den Fluidbypass 50 in den Füllorganinnenraum 12 strömen kann. Entsprechend herrscht in dem Füllorganinnenraum 12 ein Unterdruck, welcher verhindert, dass Fluid beziehungsweise Füllprodukt aus dem Füllorganinnenraum 12 durch den Fluidbypass 50 aus dem Füllorgan 10 austritt.

Zur Sterilisierung des Füllorgans 10 wird der Ventilkegel 40 in den Ventilsitz 30 abdichtend abgesenkt. Anschließend wird der Füllorganinnenraum 12 mit einem Sterilisationsmedium, wie zum Beispiel Sattdampf, beaufschlagt. Dabei im Füllorganinnenraum 12 entstehendes Kondensat kann durch den Fluidbypass 50 abfließen. Ferner kann ein dampf- beziehungsweise gasförmiger Teil des Sterilisationsmediums über den Fluidbypass 50 aus dem Füllorganinnenraum 12 ausströmen. Der ausströmende Dampf kann über entsprechende Sensoren im Bereich der Produktabgabeöffnung 14 gemessen werden, um somit Rückschlüsse über den Ablauf der Sterilisierung im Füllorganinnenraum 12 treffen zu können. Dabei kann insbesondere die Temperatur und der Druck des Sterilisationsmediums im Füllorganinnenraum 12 überwacht werden.

Um auch die Dichtkontaktflächen 32, 42 des Ventilsitzes 30 und des Ventilkegels 40 zu sterilisieren, kann der Ventilkegel 40 während der Sterilisierung des Füllorganinnenraumes 12 aus dem Ventilsitz 30 herausgehoben werden. Das Sterilisierungsmedium strömt dann zwischen dem Ventilkegel 40 und dem Ventilsitz 30 über die Dichtkontaktoberflächen 32, 42 hinweg zur Produktabgabeöffnung 14.

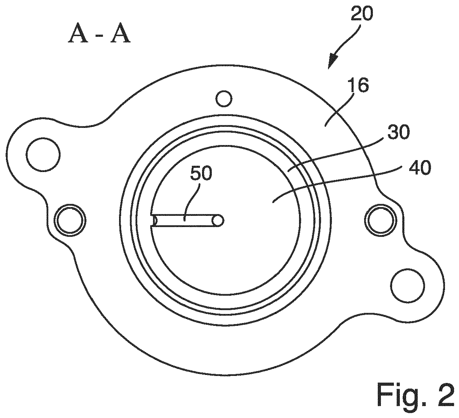

Figur 2 ist eine Schnittansicht des Füllventils 20 entlang des in Figur 1 gezeigten Schnitts A-A zu entnehmen. Die horizontale Bohrung des Fluidbypasses 50 verläuft von dem Rand des Ventilkegels 40 hin zur Mitte des Ventilkegels 40. In der Mitte des Ventilkegels 40 trifft die horizontale Bohrung des Fluidbypasses 50 auf eine senkrechte Bohrung. Der Ventilkegel 40 und der Ventilsitz 30 sind aus Edelstahl gefertigt. Alternativ können der Ventilkegel 40 und der Ventilsitz 30 auch aus anderen Materialien gefertigt sein.

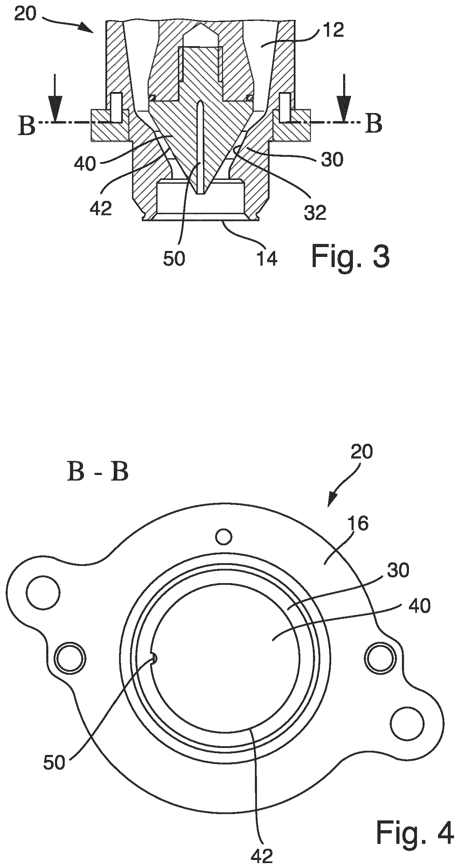

Figur 3 ist ein Füllventil 20 zu entnehmen, welches sich von den in den Figuren 1 und 2 gezeigten Füllventilen dadurch unterscheidet, dass der Fluidbypass 50 in Form einer Nut mit abgerundetem Boden auf der Dichtkontaktoberfläche 42 des Ventilkegels 40 angeordnet ist. In Figur 3 ist der Ventilkegel 40 geringfügig aus dem Ventilsitz 30 herausgehoben. Der Spalt zwischen dem Ventilkegel 40 und dem Ventilsitz 30 ist dabei mit dem durch die Nut mit abgerundetem Boden, welche den Fluidbypass 50 bildet, vereint. Entsprechend weist das Füllventil 20 in diesem Zustand keine zusätzliche Überbrückung auf, welche den Füllorganinnenraum 12 mit der Produktabgabeöffnung 14 verbindet. Die Überbrückung wird vielmehr erst dann bereitgestellt, wenn der Ventilkegel 40 abdichtend auf den Ventilsitz 30 abgesenkt ist. In diesem Zustand ist der Füllorganinnenraum 12 mit der Produktabgabeöffnung 14 über den Fluidbypass 50 verbunden. Die Nut mit abgerundetem Boden kann beispielsweise im Fräsverfahren auf dem Ventilkegel 40 erzeugt werden.

Figur 4 zeigt eine Schnittansicht des Füllventils 20 entlang des in Figur 3 gezeigten Schnitts B-B. Dabei weist der Ventilkegel 40 auf der Dichtkontaktoberfläche 42 den Fluidbypass 50 in Form einer Nut mit abgerundetem Boden auf.

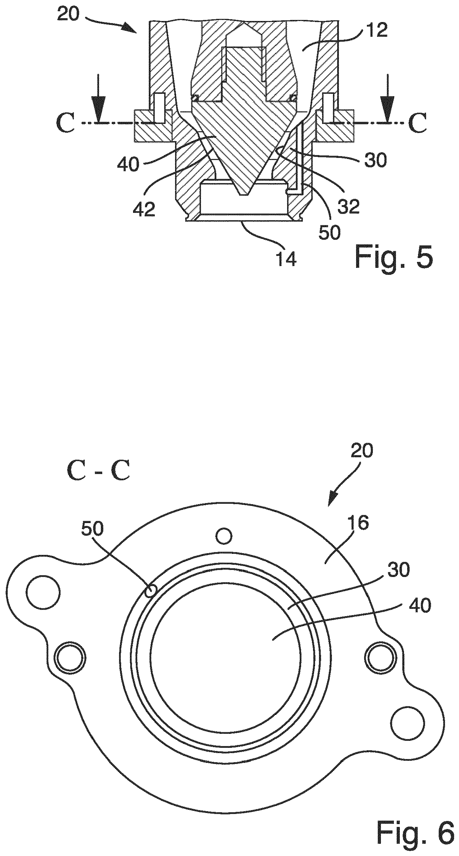

Figur 5 ist ein Füllventil 20 zu entnehmen, wobei der Fluidbypass 50 im Ventilsitz 30 angeordnet ist. Der Fluidbypass 50 wird durch eine horizontale Bohrung und eine senkrechte Bohrung im Ventilsitz 30 bereitgestellt. Dabei erstreckt sich die senkrechte Bohrung vom Füllorganinnenraum 12 nach unten und trifft auf die horizontale Bohrung, welche in die Produktabgabeöffnung 14 mündet. Ist der Ventilkegel 40 abdichtend auf den Ventilsitz 30 abgesenkt, kann die Abdichtung mittels des Fluidbypasses 50 überbrückt werden.

Figur 6 ist eine Schnittansicht des Füllventils 20 entlang des in Figur 5 gezeigten Schnitts C-C zu entnehmen. Der Fluidbypass 50 ist im Ventilsitz 30 angeordnet und mündet in diesen über eine senkrechte Bohrung.

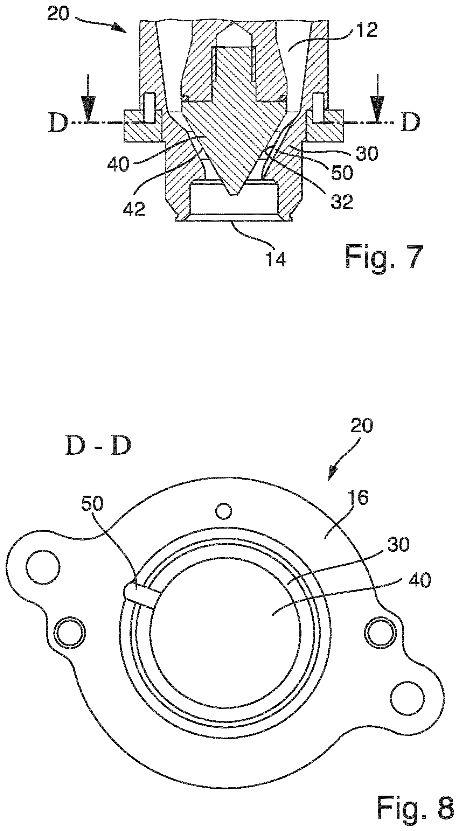

Figur 7 ist ein Füllventil 20 zu entnehmen, wobei der Fluidbypass 50 in Form einer Aussparung in der Dichtkontaktoberfläche 32 des Ventilsitzes 30 angeordnet ist. Wird der Ventilkegel 40 abdichtend in den Ventilsitz 30 abgesenkt, verbindet der Fluidbypass 50 den Füllorganinnenraum 12 mit der Produktabgabeöffnung 14. Während einer Sterilisierung des Füllorgans kann so Kondensat und Dampf über den Fluidbypass 50 aus dem Füllorganinnenraum 12 austreten, während der Ventilkegel 40 abdichtend in den Ventilsitz 30 abgesenkt ist.

Figur 8 ist eine Schnittansicht des Füllventils 20 entlang des in Figur 7 gezeigten Schnitts D-D zu entnehmen. Auf der Oberfläche des Ventilsitzes 30 ist der Fluidbypass 50 in Form einer Aussparung angeordnet.

### Bezugszeichenliste

- 10: Füllorgan
- 12: Füllorganinnenraum
- 14: Produktabgabeöffnung
- 16: Gehäuse
- 20: Füllventil
- 22: Feder
- 24: Ventilstange
- 25: Führung
- 26: Hubzylinder
- 27: Feder
- 28: Druckluftkanal
- 30: Ventilsitz
- 32: Dichtkontaktoberfläche
- 40: Ventilkegel
- 42: Dichtkontaktoberfläche
- 50: Fluidbypass
- 60: Füllproduktzulauf
- 62: Füllproduktzulaufventil

## Patentansprüche

1. Füllventil (20) für einen Füller zum Befüllen eines Behälters mit einem Füllprodukt, bevorzugt für eine Getränkeabfüllanlage, umfassend einen Ventilsitz (30), einen dazu komplementären Ventilkegel (40), der zur Bereitstellung einer Abdichtung in den Ventilsitz (30) absenkbar ist, und einen Fluidbypass (50) zur Überbrückung der durch die Absenkung des Ventilkegels (40) in den Ventilsitz (30) bereitgestellten Abdichtung für ein Fluid,
**dadurch gekennzeichnet, dass**
ein Füllproduktzulaufventil (62) stromaufwärts des Ventilsitzes (30) angeordnet ist, das eingerichtet ist, um einen Zulauf von Füllprodukt zu dem Ventilsitz (30) im Füllbetrieb zu unterbrechen.

2. Füllventil (20) gemäß Anspruch 1, **dadurch gekennzeichnet, dass** der Ventilsitz (30) zwischen einem Füllorganinnenraum (12) und einer Produktabgabeöffnung (14) angeordnet ist und der Fluidbypass (50) den Füllorganinnenraum (12) bei abdichtend abgesenktem Ventilkegel (40) mit der Produktabgabeöffnung (14) verbindet.

3. Füllventil (20) gemäß Anspruch 1 oder 2, **dadurch gekennzeichnet, dass** der Fluidbypass (50) die Form einer Bohrung aufweist.

4. Füllventil (20) gemäß einem der vorhergehenden Ansprüche, **dadurch gekennzeichnet, dass** der Fluidbypass (50) einen Durchmesser, Querschnitt oder Breite von 0,1 mm bis 5 mm, bevorzugt 0,5 mm bis 4 mm, und besonders bevorzugt 1 mm bis 3 mm, aufweist.

5. Füllventil (20) gemäß einem der vorhergehenden Ansprüche, **dadurch gekennzeichnet, dass** der Fluidbypass (50) in dem Ventilkegel (40) angeordnet ist.

6. Füllventil (20) gemäß Anspruch 5, **dadurch gekennzeichnet, dass** der Fluidbypass (50) auf einer Dichtkontaktoberfläche (42) des Ventilkegels (40) in Form einer Aussparung, bevorzugt einer Nut, und besonders bevorzugt einer Nut mit einem abgerundeten Boden, angeordnet ist.

7. Füllventil (20) gemäß einem der Ansprüche 1 bis 4, **dadurch gekennzeichnet, dass** der Fluidbypass (50) in dem Ventilsitz (30) angeordnet ist, wobei bevorzugt der Fluidbypass (50) auf einer Dichtkontaktoberfläche (32) des Ventilsitzes (30) in Form einer Aussparung, bevorzugt einer Nut, und besonders bevorzugt einer Nut mit einem abgerundeten Boden, angeordnet ist.

8. Füllventil (20) gemäß einem der vorstehenden Ansprüche, **dadurch gekennzeichnet, dass** das Füllproduktzulaufventil (62) stromaufwärts des Ventilsitzes (30) einen Zulauf von Füllprodukt zu dem Ventilsitz (30) im Füllbetrieb taktweise unterbindet.

9. Verfahren zum Sterilisieren eines Füllers, welcher mindestens ein Füllventil (20) gemäß einem der Ansprüche 1 bis 8 umfasst, mit den Schritten:
Absenken des Ventilkegels (40) auf den Ventilsitz (30), und
Beaufschlagen des Füllers, bevorzugt eines Füllorganinnenraums (12), mit einem Sterilisationsmedium, bevorzugt Heißdampf, zur Sterilisierung,
**dadurch gekennzeichnet, dass**
ein Teil des Sterilisationsmediums während der Sterilisierung des Füllers durch den Fluidbypass (50) die durch die Absenkung des Ventilkegels (40) in den Ventilsitz (30) bereitgestellte Abdichtung überbrückt, und im Füllbetrieb ein Zulauf von Füllprodukt bei in den Ventilsitz (30) abgesenktem Ventilkegel (40) durch das Füllproduktzulaufventil (62) unterbrochen wird.

10. Verfahren gemäß Anspruch 9, **dadurch gekennzeichnet, dass** das Sterilisationsmedium unter einem Druck von 1,5 bar bis 5,5 bar, bevorzugt 2 bar bis 5 bar, besonders bevorzugt 3 bar bis 4 bar, und ganz besonders bevorzugt 3,5 bar bei einer Temperatur zwischen 100 °C und 150 °C, bevorzugt 121 °C bis 135 °C, bereitgestellt wird.

11. Verfahren gemäß Anspruch 9 oder 10, **dadurch gekennzeichnet, dass** der Ventilkegel (40) zum Sterilisieren von Dichtkontaktoberflächen des Ventilsitzes (30) und des Ventilkegels (40) aus dem Ventilsitz (30) herausgehoben, bevorzugt periodisch oder taktweise herausgehoben, wird und beim Eintreten eines vorbestimmten Ereignisses wieder in den Ventilsitz (30) abgesenkt wird.

12. Verfahren gemäß Anspruch 11, **dadurch gekennzeichnet, dass** das vorbestimmte Ereignis das Ablaufen einer vorbestimmten Zeit und/oder das Erreichen einer vorbestimmten Temperatur der Dichtkontaktoberfläche (32) des Ventilsitzes (30) und/oder der Dichtkontaktoberfläche (42) des Ventilkegels (40) ist.

13. Verfahren gemäß einem der Ansprüche 9 bis 12, **dadurch gekennzeichnet, dass** der Ventilkegel (40) zum Sterilisieren der Dichtkontaktoberfläche (32) des Ventilsitzes (30) und der Dichtkontaktoberfläche (42) des Ventilkegels (40) um 1 mm bis 3 mm aus dem Ventilsitz (30) angehoben wird.

## Claims

1. Filling valve (20) for a filler for filling a container with a filling product, preferably for a drinks filling installation, comprising a valve seat (30), a valve cone (40), which is formed complementary to the valve seat (30) and can be lowered into the valve seat (30) to provide a seal, and a fluid bypass (50) for bridging the seal which is provided by the lowering of the valve cone (40) into the valve seat (30) for a fluid, **characterised in that** there is arranged upstream of the valve seat (30) a filling product supply valve (62) which is configured to interrupt a supply of filling product to the valve seat (30) during filling operation.

2. Filling valve (20) according to claim 1, **characterised in that** the valve seat (30) is arranged between a filling member inner space (12) and a product dispensing opening (14), and the fluid bypass (50) connects the filling member inner space (12) to the product dispensing opening (14) when the valve cone (40) is lowered in a sealing manner.

3. Filling valve (20) according to claim 1 or 2, **characterised in that** the fluid bypass (50) is in the form of a hole.

4. Filling valve (20) according to any one of the preceding claims, **characterised in that** the fluid bypass (50) comprises a diameter, cross-section or width of from 0.1 mm to 5 mm, preferably from 0.5 mm to 4 mm, and in a particularly preferred manner from 1 mm to 3 mm.

5. Filling valve (20) according to any one of the preceding claims, **characterised in that** the fluid bypass (50) is arranged in the valve cone (40).

6. Filling valve (20) according to claim 5, **characterised in that** the fluid bypass (50) is arranged on a sealing contact surface (42) of the valve cone (40) in the form of a recess, preferably a groove, and in a particularly preferred manner a groove with a rounded base.

7. Filling valve (20) according to any one of claims 1 to 4, **characterised in that** the fluid bypass (50) is arranged in the valve seat (30), wherein the fluid bypass (50) is preferably arranged on a sealing contact surface (32) of the valve seat (30) in the form of a recess, preferably a groove, and in a particularly preferred manner a groove with a rounded base.

8. Filling valve (20) according to any one of the preceding claims, **characterised in that** the filling product supply valve (62) upstream of the valve seat (30) prevents in a timed manner a supply of filling product to the valve seat (30) during filling operation.

9. Method for sterilising a filler which comprises at least one filling valve (20) according to any one of claims 1 to 8, comprising the steps of:
lowering the valve cone (40) onto the valve seat (30), and
applying a sterilisation medium, preferably hot steam, on the filler, preferably a filling member inner space (12), for sterilisation,
**characterised in that**
a portion of the sterilisation medium during the sterilisation of the filler by the fluid bypass (50) bridges the seal which is provided by the lowering of the valve cone (40) into the valve seat (30), and during filling operation a supply of filling product is interrupted by the filling product supply valve (62) when the valve cone (40) is lowered into the valve seat (30).

10. Method according to claim 9, **characterised in that** the sterilisation medium is provided at a pressure of from 1.5 bar to 5.5 bar, preferably from 2 bar to 5 bar, in a particularly preferred manner from 3 bar to 4 bar, and in a quite particularly preferred manner 3.5 bar at a temperature between 100°C and 150°C, preferably from 121°Cto 135°C.

11. Method according to claim 9 or 10, **characterised in that** the valve cone (40) in order to sterilise sealing contact surfaces of the valve seat (30) and the valve cone (40) is lifted out of the valve seat (30), preferably lifted out in a periodic or timed manner, and, when a predetermined event occurs, is lowered into the valve seat (30) again.

12. Method according to claim 11, **characterised in that** the predetermined event is the elapsing of a predetermined time and/or reaching a predetermined temperature of the sealing contact surface (32) of the valve seat (30) and/or the sealing contact surface (42) of the valve cone (40).

13. Method according to any one of claims 9 to 12, **characterised in that** the valve cone (40) in order to sterilise the sealing contact surface (32) of the valve seat (30) and the sealing contact surface (42) of the valve cone (40) is raised out of the valve seat (30) by from 1 mm to 3mm.

## Revendications

1. Soupape de remplissage (20) pour un dispositif de remplissage afin de remplir un récipient par un produit de remplissage, de préférence pour une installation de remplissage de boisson, comprenant un siège de soupape (30), une cône de soupape (40) qui lui est complémentaire au siège de soupape (30) et qui peut être abaissé dans le siège de soupape (30) pour mettre en place un joint étanche, et une dérivation de fluide (50) pour ponter le joint étanche mis en place par l'abaissement du cône de soupape (40) dans le siège de soupape (30) pour un fluide,
**caractérisée en ce que**
une soupape d'alimentation en produit de remplissage (62) est agencée en amont du siège de soupape (30), qui est aménagée pour interrompre une alimentation de produit de remplissage au siège de soupape (30) en plein fonctionnement de remplissage.

2. Soupape de remplissage (20) selon la revendication 1, **caractérisée en ce que** le siège de soupape (30) est agencé entre un espace interne d'organe de remplissage (12) et une ouverture de décharge de produit (14) et la dérivation de fluide (50) raccorde l'espace interne d'organe de remplissage (12) à l'ouverture de décharge de produit (14) lorsque le cône de soupape (40) est abaissé de manière étanche.

3. Soupape de remplissage (20) selon la revendication 1 ou 2, **caractérisée en ce que** la dérivation de fluide (50) présente la forme d'un alésage.

4. Soupape de remplissage (20) selon l'une quelconque des revendications précédentes, **caractérisée en ce que** la dérivation de fluide (50) présente un diamètre, une section transversale ou une largeur de 0,1 mm à 5 mm, de préférence de 0,5 mm à 4 mm, plus particulièrement de 1 mm à 3 mm.

5. Soupape de remplissage (20) selon l'une quelconque des revendications précédentes, **caractérisée en ce que** la dérivation de fluide (50) est agencée dans le cône de soupape (40).

6. Soupape de remplissage (20) selon la revendication 5, **caractérisée en ce que** la dérivation de fluide (50) est agencée sur une surface de contact étanche (42) du cône de soupape (40) sous la forme d'une cavité, de préférence une rainure, en particulier une rainure avec un fond arrondi.

7. Soupape de remplissage (20) selon l'une quelconque des revendications 1 à 4, **caractérisée en ce que** la dérivation de fluide (50) est agencée dans le siège de soupape (30), dans laquelle, de préférence, la dérivation de fluide (50) est agencée sur une surface de contact étanche (32) du siège de soupape (30) sous la forme d'une cavité, de préférence d'une rainure et en particulier d'une rainure avec un fond arrondi.

8. Soupape de remplissage (20) selon l'une quelconque des revendications précédentes, **caractérisée en ce que** la soupape d'alimentation en produit de remplissage (62) en amont du siège de soupape (30) interrompt de manière intermittente en plein fonctionnement de remplissage une alimentation du produit de remplissage dans le siège de soupape (30).

9. Procédé de stérilisation d'un dispositif de remplissage, qui comprend au moins une soupape de remplissage (20) selon l'une quelconque des revendications 1 à 8, avec les étapes:
abaisser le cône de soupape (40) sur le siège de soupape (30) et
alimenter le dispositif de remplissage, de préférence un espace interne d'organe de remplissage (12), par un agent de stérilisation, de préférence de la vapeur chaude, pour effectuer le stérilisation,
**caractérisé en ce que :**
une partie de l'agent de stérilisation ponte, au cours de la stérilisation du dispositif de remplissage via la dérivation de fluide (50), le joint étanche aménagé par l'abaissement du cône de soupape (40) dans le siège de soupape (30) et, en plein fonctionnement de remplissage, une alimentation en produit de remplissage par la soupape d'alimentation en produit de remplissage (62) est interrompue lorsque le cône de soupape (40) est abaissé dans le siège de soupape (30).

10. Procédé selon la revendication 9, **caractérisé en ce que** l'agent de stérilisation est placé sous une pression de 1,5 bar à 5,5 bars, de préférence de 2 bars à 5 bars, mieux encore de 3 bars à 4 bars et bien mieux encore de 3,5 bars à une température entre 100 °C et 150 °C, de préférence de 121 °C à 135 °C.

11. Procédé selon la revendication 9 ou 10, **caractérisé en ce que** le cône de soupape (40) est soulevé du siège de soupape (30) pour stériliser les surfaces de contact étanches du siège de soupape (30) et du cône de soupape (40), de préférence soulevé périodiquement ou de manière intermittente, et est abaissé à nouveau dans le siège de soupape (30) lorsque se produit un événement prédéterminé.

12. Procédé selon la revendication 11, **caractérisé en ce que** l'événement prédéterminé est l'écoulement d'une période prédéterminée et/ou l'avènement d'une température prédéterminée de la surface de contact étanche (32) du siège de soupape (30) et/ou de la surface de contact étanche (42) du cône de soupape (40).

13. Procédé selon l'une quelconque des revendications 9 à 12, **caractérisé en ce que** le cône de soupape (40) est soulevé du siège de soupape (30) de 1 mm à 3 mm pour stériliser la surface de contact étanche (32) du siège de soupape (30) et la surface de contact étanche (42) du cône de soupape (40).
